# EUROPEAN PATENT SPECIFICATION

(11) **EP 0 293 256 B1**
(45) Date of publication and mention of the grant of the patent: **11.09.1996**
(21) Application number: 88304881.1
(22) Date of filing: 27.05.1988
(51) Int. Cl.: A61F 2/12, B29C 43/02

(54) **Method of producing textured surface prosthesis implants**
Methode zur Herstellung eines Prothesenimplantats mit Texturoberfläche
Méthode pour la fabrication d'un Implant prothétique comprenant une surface texturée

(30) Priority: 27.05.1987 US 54607
(43) Date of publication of application: 30.11.1988
(73) Proprietor: MENTOR CORPORATION, Goleta California 93117 (US)
(72) Inventor: Yan, John Y.J., Santa Barbara California 91301 (US); Purkait, Bobby K., Santa Barbara California 93103 (US)
(74) Representative: Thomson, Paul Anthony

(56) References cited:
- EP-A- 0 174 141
- FR-A- 2 199 266
- GB-A- 2 021 954
- US-A- 3 366 975
- US-A- 3 683 424
- US-A- 4 531 244
- US-A- 4 557 779

## Description

This invention relates to a process for making a textured surgical implant, such as a breast implant, having a textured non-porous exterior surface thereon.

### BACKGROUND OF THE INVENTION

It is well-known in the field of plastic surgery to enlarge the female breast or replace other body organs or structures with prostheses which are surgically implanted therein. With respect to female breasts, in many instances it becomes necessary to remove the entire mammary gland or a substantial portion thereof as a result of cancerous infection or other disease. The surgical removal of the diseased body tissue leaves voids which may be filled by such a prosthetic implant. Such implants provide physical support for the surrounding body tissue and organs and in the case of voids near the skin, preserve the outward appearance of the body. When cancerous, pre-cancerous, or damaged tissue is removed, it is often possible to insert the prosthesis to be implanted through the same surgical incision used for removing the tissue. Particularly in the cases where a radical removal of tissue has occurred it is desirable to use an implant for the purpose of restoring the human body to its original normal form. The restoration of the normal appearance of the body has an extremely beneficial phychological effect on post-operative patients, eliminating much of the shock and depression that often follows extensive surgical procedures.

Among the various problems involved in prosthetic implants are those of preserving the natural shape of the body over time after implantation. Some prior art implants comprise an outer layer of sponge material which retains a relatively natural softness and resiliency similar to the replaced body tissue. However, the inherent porosity of such sponge material has also been a source of considerable inconvenience and disappointment because the sponge eventually becomes invaded with connective tissue, sometimes called capsule formation, throughout all or a major portion of the implant. The connective tissue, being fiberous, shrinks as it ages, sometimes called capsular contraction, resulting in the compression of the implant thereby causing the implant to lose both its original size and shape and its original resiliency. Cases are known where shrinkage has been as much as 20 to 30% of the original size. These changes in shape, size and resiliency may be distressing and embarrassing to the patient and are obviously undesirable.

Other prior art prosthesis comprise a smooth outer surface so that there can be no infiltration of the fibroblasts into the surface of the prosthesis. However, it is difficult to obtain anchoring of the prosthesis to the body because of the difficulty of the connective tissue grown thereon to form a satisfactory attachment thereto. Also, these smooth surface prostheses which are covered with a cellular layer which can not become infiltrated by living tissue, but instead, have a more or less external surface which is formed of an impervious material, so that body fluid can and often does accumulate between the prosthesis and the living tissue. This fluid accumulation may be directly or indirectly related to infection in the patient at the site of the fluid accumulation. Also, in severe cases, the infection may cause necrosis of the tissue in the location of the prosthesis.

Another type of breast prosthesis which is known in the art has a smooth surface to which is glued a porous polyurethane foam or Dacron velour material. It has been suggested that these porous surfaces allow the penetration of tissue and fluids from the surrounding tissue. It has also been suggested that such polyurethane coated prosthesis inhibit the problems of capsular contraction. (See U.S. Patent No. 4,648,880 and Pennisi, Aesth. Plast. Surg. 9:73-77, 1985). These porous surface prostheses are thick and not very compliant, and therefore, in some cases, are difficult to insert beneath the skin with a small surgical incision. As a result, large incisions which cause unsightly scars may be necessary in order to provide a sufficiently large hole for proper insertion. These porous prostheses are also absorbant to bacterial containing body fluids and thus make infection control difficult. In addition, if the porous material is polyurethane foam, there have been a number of reports that the foam disintegrates in the body after a few months causing severe rashes. Also marked foreign body reaction may also be observed with these foam implants. (Plastic and Reconst. Surg. 61, No. 1, 1978.) Rashes have also been known to occur soon after implantation in some instances, possibly as a result of a body reaction to the antibiotic solution in which the polyurethane coated prosthesis is dipped prior to implantation.

In the standard process for manufacturing surgical prosthesis, and particularly, mammary implants, a shell of desired thickness is formed having the desired shape for the particular implant and purpose. These shells may be single lumen, multilumen or expendable type prosthesis such as those used for tissue expansion. The shell generally has a circular hole, termed a patch hole. The patch hole is then covered with a patch which is attached thereto using silicone rubber or other similar biocompatible adhesive. The prosthesis is then filled through a small fill hole with saline, gel foam, combinations of these materials or other suitable material known in the art and the fill hole is sealed. The prosthesis, having a smooth exterior, is then sterilized and implanted, or it may be covered with a foam material as described above prior to sterilization and implantation.

U.S. Patent No. 4,531,244 relates to a mammary prosthesis having protruberances on the outer surface thereof. However, such prosthesis is not able to accomplish the stated purpose of preventing the formation of a hardened capsule around the implant. The protruberances in such prior art device are so large that they would not effectively inhibit fibroblast growth

In accordance with the present invention there is provided a process for making a textured surgical implant comprising:
providing a smooth surface prosthesis shell having an exterior surface;
disposing said shell on a mounting means;
disposing a formable, biocompatible material over said shell at least over a portion of said exterior surface;
providing a texturizing means;
disposing said texturizing means over said material thereby imprinting in said material a textured surface;
removing said texturing means from said material;
curing said material into said shell;
patching said shell;
filling said shell with a filler material, and
patching said filled shell to retain said filler material in said shell.

The present invention comprises a process for making a surgical prosthesis for use in augmentation mammaplasty and reconstructive surgery, having an exterior surface that is partially or fully textured. The texture surface preferably consists of a plurality of substantially microscopic peaks and valleys substantially free of pores or interstices. The textured surface permits the infiltration of tissue and fluids to enhance anchoring of the prosthesis in the body; however, the lack of pores and interstices prevent pooling or sequestration of body fluids which may otherwise increase the risk of infection. The textured surface is made of silicone rubber or other similar plastic material which is non-absorbent to body fluids, thereby inhibiting otherwise uncontrollable infection.

The textured surface may directly or indirectly disrupt capsule formation around the prosthesis, permit anchorage of tissue to the prosthesis to prevent movement at the tissue-prosthetic interface and eliminate space at the interface which can lead to bacterial infection and possibly tissue necrosis. These factors may further directly or indirectly lower or eliminate capsular contracture incidence which has been observed with prior art surgical prosthesis.

The surgical prosthesis may be used as mammary implants or other body implants such as penile prosthesis, and maxillofacial prosthesis, and for a tissue expander. The shell of the prosthesis may be single or multilumen or an expandable type and may be of any size and shape.

In the process of the invention a formed shell having a smooth exterior surface is disposed on a flattened or slightly curved disk and is coated on its exterior surface on one or both sides of the disk with unvulcanized or partially vulcanized silicone. The silicone layer is then covered with a porous or textured medium and the entire combination is compressed between platens for a short time until a textured surface is formed in the silicone layer. The texturized shell is then cured. After curing, the patch hole is covered with a patch which may or may not be texturized utilizing a similar procedure.

In an embodiment, the shell is tightly wrapped around a mandrel having a corresponding shape. The shell is then coated with an unvulcanized or partially vulcanized silicone coating which is thereafter coated with a porous or textured medium having a similar shape. A form fitting press may then be applied thereto or direct pressure from the porous medium may be applied to form a textured surface on the silicone coating. The prosthesis may then be cured on or off the mandrel and patched as aforesaid.

The present invention will be further illustrated, by way of example, with reference to the accompanying drawings. It is to be understood that the invention relates to a process for making a textured surgical implant and the drawings relating to the prothesis are included for a better understanding of the invention.

Figure 1 illustrates a perspective view of a texturized mammary prosthesis.

Figure 2 illustrates an exploded view of one process for manufacturing the texturized prosthesis utilizing a flat disk.

Figure 3 illustrates another embodiment of the process for manufacturing the prosthesis utilizing a mandrel.

Figure 4 illustrates a process for manufacturing a patch for the prosthesis.

Figure 5 illustrates a texturized patch for the prosthesis.

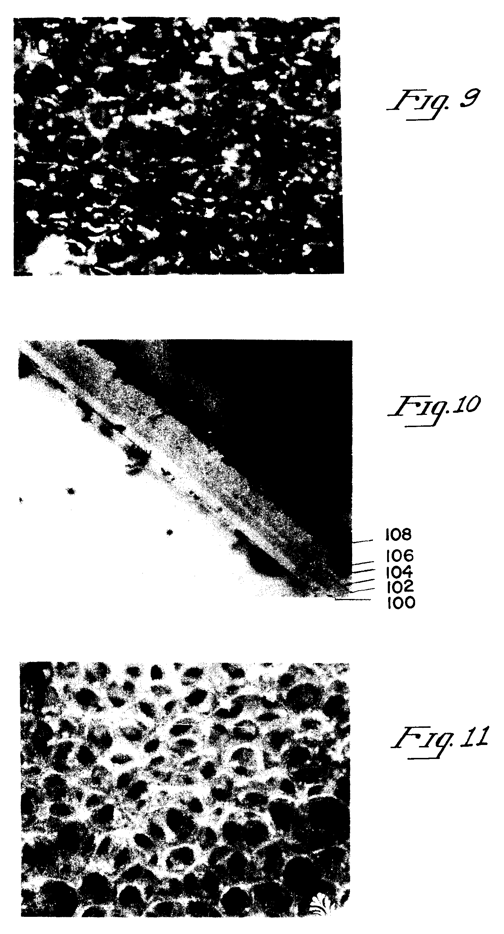

Figure 6 illustrates another embodiment for manufacturing the prosthesis utilizing a textured mandrel.

Figure 7 illustrates a sectional view of the patching of a shell of a textured prosthesis.

Figure 8 illustrates an injection mold for molding a texturized prosthesis.

Figure 9 is a 64X magnification of microscopic view of a texturized prosthesis surface (top view).

Figure 10 is a 50X magnification of a microscopic view of a texturized prosthesis (cross-sectional view).

Figure 11 is a 50X magnification of a microscopic view of a 200 micron pore size reticulated foam which may be used as a textured medium to texture the surface of the prosthesis.

The present invention is directed to a process for making a surgical prosthesis for implantation in a body for mammary, urological, gastrointestinal, or other organo-systems in which either a portion of the body has been removed or augmentation thereof is required or desired.

Whilst the present invention is directed to a process for making mammary implants, the presently described processes are suitable for making other surgical implants as well. It will be understood by a person of ordinary skill in the art that in describing the invented process with reference to mammary implants, we intend to include within the scope of the invention the process for making all surgical implants of the type wherein a textured surface may be useful.

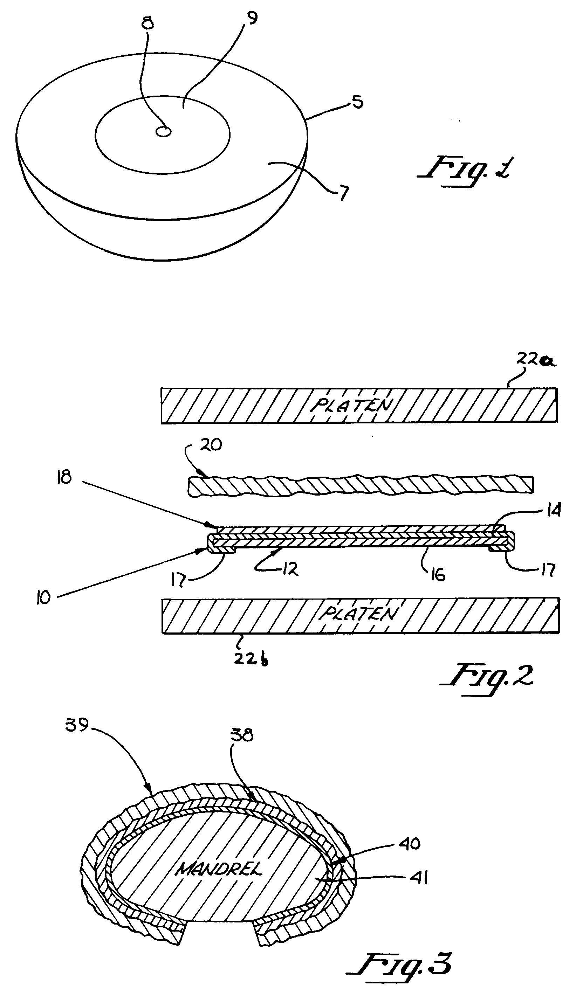

Figure 1 shows a mammary implant 5 having a completely texturized outer surface 7, a patch 9 and a fill hole 8. Of course, other shapes for the implants can be used, such as for implantation for partial mastectomies and for augmentation surgery. It will also be appreciated that while the description herein generally relates to prosthesis having a textured surface around its entire outer surface, a partial covering of the outer surface with a textured coating or topography is also contemplated.

As mentioned above, the textured, non-porous coating on the exterior surface permits greater and faster adhesion of the implant to the body by providing stronger attachment sites of the fibroblast connective tissue formed by the body in response to the implant. In addition dead space is eliminated or minimized relative to smooth surface prosthesis, thereby minimizing friction irritation. Capsular formation and the potential resulting undesirable effects of capsular contraction may also be reduced.

As used herein, the term "textured" or "texturized" used in conjunction with the present invention means a surface having minute indentations, deformations and/or raised portions on the subject surface. The width of each individual raised portion or indentation ranges in size from 0.0003 to 0.10 inches ( 0.0077 to 2.5 mm). The height of each individual indentation or raised portion is 0.0003 to 0.030 inches (0.0077 to 0.77 mm). Thus, the general appearance of the prosthesis is that of an opaque surface, slightly rough to the touch.

An important feature of the textured surface is the lack or substantial lack of pores or interstices which can accumulate or sequester body fluids and provide a volume in which infection can proliferate. The textured surface has raised portions or indentations which are generally transverse to the plane defined by the surface. The projections on the surface of the textured prosthesis may be generally columnar if a screen with round perforations therein is used as the textured medium. The projections may be irregularly shaped if a foam material is used as a texturized medium. The projections or indentations may be regular geometric shapes if a screen with a crossed or knitted pattern is used to texturize the surface. The essential difference between the present invention and prior art textured prosthesis utilizing woven, knitted, braided or felt fiber coatings on the exterior thereof, such as Dacron (Resistered Trade Mark) velour, is that the present invention generally lacks pores or interstices disposed in a direction primarily parallel to the surface of the prosthesis. Fiber materials, on the other hand, tend to have such pores or interstices parallel to the surface which can provide pockets capable of sequesting the body fluid.

As used herein, the term "absorbent" means a material itself, or a material having a particular structure making it capable of retaining a liquid by any of a number of different mechanisms. For example, some materials such as paper and cotton, absorb liquid into the individual fibers comprising such materials. Other materials may be comprised of fibers which are not of themselves absorbent, but which absorb fluid by capillary action diffusion or similar physical properties as a result of their structure. For example, the polyurethane fiber forming a foam coating disclosed by Pangman U.S. Patent Nos. 3,366,975 and 3,683,424, are described as having non-absorbent fibers. However, the foam materials of Pangman are very absorbent as a result of liquid being drawn into the pores and interstices of the foam by diffusion or capillary action. The present invention comprises a substantially non-absorbent coating, as the word "absorbent" is used herein.

Figure 2 illustrates an exploded view of a portion of one method of manufacturing the prosthesis. As shown in Figure 2, an unpatched surgical prosthesis 10, such as for use as a mammary implant, is stretched over a flat or low curvature disk 12 having a circular, oval or other suitable shaped cross-section. The majority of the exterior surface of the prosthesis is located on the upper side 14 of the disk 12. A layer or multiple layers of unvulcanized or partially vulcanized silicone 18 with a total thickness of 0.003 inches to 0.10 inches (0.0077 to 2.5 mm) covers the upper surface 14 of the prosthesis. Only a small portion 17 of the prosthesis 10 is disposed on a lower surface 16 of the disk 12. In this configuration the silicone covering is disposed across almost the entire exterior surface of the prosthesis such that no seam will appear visible at the top or substantially any of the sides of the finished prosthesis.

In the next step of the first process disclosed herein, the silicone covering 18 is covered with a porous or textured medium 20. This porous or textured medium 20 may be, for example, foam, a perforated screen or a specially molded form having a textured surface of the particular desired design and topography. In the presently preferred embodiment the medium comprises reticulated foam having approximately 100 pores per 2.54 cm (inch). The entire assembly including the disk 12, prosthesis 10, silicone layer 18 and porous or textured medium 20 is then compressed using either cold or hot compressive platens 22a and 22b. The use of hot platens in the compression of silicone and similar materials is well known in the art. If cold platens are used, the silicone must be carefully handled until it is vulcanized, or partially vulcanized. In the presently preferred embodiment, cold platens are used having a compressive force of 552 kPa (80 pounds per square inch) for 0.5 to 2.5 minutes.

After compression, the platens 22a and 22b are removed and the medium 20 is also removed leaving a texturized imprint in the silicone layer 18. The prosthesis 10 with the imprinted texturized silicone layer 18 is then removed from the disk 12 and the prosthesis with the imprinted silicone layer is cured at vulcanizing temperatures, as is known in the art. In particular, in the preferred embodiment, curing takes place at approximately 176,7° to 204,4° C (350° to 400° F) over a period of four hours, although the particular temperature and time may vary depending upon the particular materials used. In the preferred embodiment, the silicone material is obtained from Dow Corning.

In the preferred embodiment, shown in Figure 7, the patch is textured and adhered to the shell in a single step. As shown, a textured shell 60 is provided, as described above. An untextured patch 62 comprising a vulcanized layer of silicone sheeting 63 and an unvulcanized layer of silicone 61 is also provided and disposed within the shell 60 with the unvulcanized layer 61 facing outward and the perimeter 65 of the patch 62 overlapping with the edge 66 of the shell 60. A textured or porous medium 67 is disposed over the patch 62 and the entire assembly is compressed between hot platens 68a and 68b at 176,7° C (350° F) and 552 kPa (80 psi) for 2.5 minutes. The platens 68a and 68b and the medium 67 is removed, and the patched shell is cured in an oven at 176,7° C (350° F) for 4 hours.

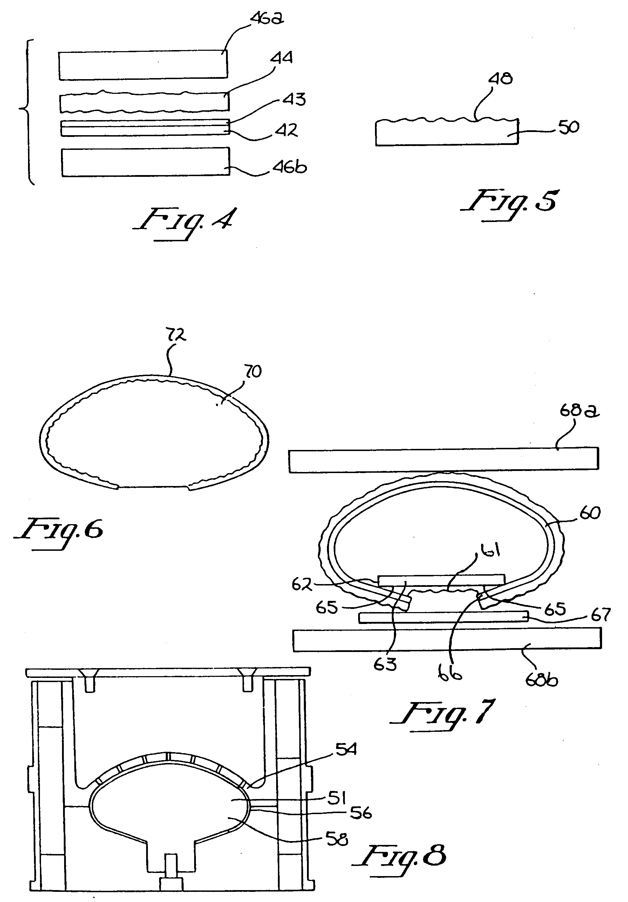

In an alternative method, the patch is preferrably texturized as shown in Figures 4 and 5 in a manner similar to the process for texturing the prosthesis shell as described above. Figure 4 is an exploded view of the process for making a textured patch. A sheet of partially vulcanized silicone sheeting 42 is formed from unvulcanized silicone, commercially available from Dow Corning calendered into sheets, vulcanized and then coated with unvulcanized silicone 43. The sheeting is covered with a porous or textured surface medium 44 of the same type as described above. The combination of sheeting 42 and medium 44 are compressed between platens 46a and 46b to form a patch 50 having a single sided texturized surface 48 as shown in Figure 5. The textured patch is preferably placed on the inside of the prosthesis with unvulcanized or partially vulcanized silicone facing outward and a washer or ring of unvulcanized silicone disposed therebetween to adhere the patch to the shell. The surface facing inward toward the inside of the prosthesis is precured so that it does not stick to the inner surface of the prosthesis shell.

Whichever process is used for texturing the patch hole, the prosthesis with the textured uncured patch hole is then cured in the same manner as discussed above, or as otherwise required by the manufacturer's instructions.

The above-described process provides a prosthesis having no seam on its exterior surface, with its entire surface being texturized. The following procedure may be used in the manufacture of a surgical prosthesis having a textured surface except that a seam or overlapping of the silicone layers on the exterior surface will be provided. In this embodiment, the prosthesis is stretched over a disk such that the outer diameter is at or near the edge of the disk. One silicone layer with a thickness of between 0.003 and 0.10 inches (0.0077 to 2.5 mm) are placed on the top and bottom sides of the disk and a porous or textured medium is placed over each silicone layer. It is not at all necessary to texturize the two sides of the surgical prosthesis at the same time or in any particular sequence. The entire prosthesis is patched in this manner as well. The entire prosthesis is then cured in accordance with the above-described process and may than be filled as discussed below by any other procedure known in the art. In this manner, in a single step, a partially textured surgical prosthesis is provided.

In yet another embodiment of the present invention as shown in Figure 3 a surgical prosthesis 40 is disposed on a mandrel 41 having a smooth exterior surface. The prosthesis 40 is then coated with unvulcanized or partially vulcanized silicone 38 coated over the surgical prosthesis 40. A porous or textured medium 39 is then fitted over the silicone layer 38 with sufficient pressure to provide therein an imprint on the silicone surface 43. The textured medium 39 can be provided over the entire surface or over a portion of the surface, as desired. The textured medium 39 is then removed from the silicone coating 38 and the prosthesis is cured at elevated temperatures or at room temperature, as desired. The prosthesis manufactured in this manner may then patched with a textured patch as described above.

After the prosthesis is manufactured having the desired textured surface it may then be filled with a fluid such as saline, gel or other resilient material as is known in the art. As discussed above, the textured surface may cover the entire exterior wall of the prosthetic body or may only partially cover the exterior surface to provide the desired characteristics. Of course, the roughness and specific surface topography of the prosthesis can be altered by the use of different textured or porous media having corresponding roughness or topography. The textured surface material and the prosthetic material may either or both be made of silicone rubber or other suitable plastic materials.

The materials which may be used to form the shell include silicone rubber, segmental polyurethane, copolymers of silicone, laminates of various forms of silicone, silicone copolymers and polyurethane, and various other elastomers alone or in combination such as those described in U.S. Patent Nos. 4,592,755 and 4,205,401. In addition, many of the foregoing materials, may be used to form the textured surface on the shell and patch.

The shell to be texturized can be any of a number of styles, sizes and shapes. It may be either single or multi-lumen. It may be of the expandable type such as those used as tissue expanders. It may be circular, oval, crescent-shaped, rectangular, cylindrical such as for penile implants, or a customized shape such as implants for maxillofacial implants.

Figure 9 shows the texturized surface of an invented prosthesis in a picture taken through a microscope with a 64X magnification. Figure 10 shows a microscopic 50X magnification of a cross-section of a texturized shell. The three layers 100, 102 and 104 all comprise three layers of the particular shell, and layers 106 and 108 are actually a single layer of textured silicone coated over the shell. The black layer 108 is actually a photographic artifact of the surface as a result of a diffusion of the light at the surface. Figure 11 is a 50X magnification of a reticulated foam which may be used as a textured medium.

## Claims

1. A process for making a textured surgical implant comprising:
providing a smooth surface prosthesis shell (10) having an exterior surface;
disposing said shell (10) on a mounting means (12);
disposing a formable, biocompatible material (18) over said shell at least over a portion of said exterior surface;
providing a texturizing means (20);
disposing said texturizing means (20) over said material (18) thereby imprinting in said material a textured surface;
removing said texturing means (20) from said material;
curing said material (18) into said shell (10);
patching said shell (10);
filling said shell (10) with a filler material, and patching said filled shell to retain said filler material in said shell.

2. A process as claimed in claim 1, characterised in that said mounting means comprises a substantially round disk (12) having a substantially flat cross section, said disk having a first side (14) and a second side (16), and wherein said shell (10) is substantially disposed on said first side (14) of said disk (12).

3. A process as claimed in claim 1 or 2, characterised in that said texturizing means (20) is disposed over said material (18) only on said first side (14).

4. A process as claimed in claim 2, characterised in that said material (18) is disposed on said shell (10) on said first side (14) and said second side (16) of said disk (12) and said texturing means (20) comprises two texturing means, one disposed on said material (18) on each side of said disk (12).

5. A process as claimed in any one of claims 1 to 4, characterised in that after said texturing means (20) is disposed on said material (18) said shell (10) is compressed between a pair of platens (22a,22b).

6. A process as claimed in claim 5, characterised in that said platens (22a,22b) are at ambient temperature.

7. A process as claimed in claim 5, characterised in that at least one of said platens is at an elevated temperature.

8. A process as claimed in any one of claims 1 to 7, characterised in that said mounting means is a mandrel (41) formed in the shape of said implant.

9. A process as claimed in any one of claims 1 to 8, characterised in that said formable, biocompatible material comprises silicone.

10. A process as claimed in claim 9, characterised in that said silicone comprises silicone sheeting.

11. A process as claimed in any one of claims 1 to 10, characterised in that said texturing means (20) comprises foam.

12. A process as claimed in claim 11, characterised in that said foam comprises reticulated foam.

13. A process as claimed in any of claims 1 to 12, characterised in that said texturizing means (20) comprises a texturized plate.

14. A process as claimed in any one of claims 1 to 12, characterised in that said texturizing means comprises a screen.

15. A process as claimed in any one of claims 1 to 14, characterised in that said patch (62) comprises texturized material.

16. A process as claimed in any one of claims 1 to 15, characterised in that said patching step comprises the steps of:
providing a patch (62) of partially vulcanised silicone;
disposing thereover a texturizing means (67);
inserting said texturized patch in a patch hole of said shell with the texturized portion thereof facing outward;
adhering said patch to said shell (60) with silicone; and
curing the patch shell.

## Patentansprüche

1. Verfahren zur Herstellung eines chirurgischen Texturimplantats, mit folgenden Schritten:
Bereitstellen einer ProthesenKapsel (10) mit glatter Oberfläche, die eine Außenfläche hat;
Anordnen der Kapsel (10) auf einer Haltevorrichtung (12);
Aufbringen eines formbaren, biologisch verträglichen Materials (18) auf zumindest einem Teil der Außenfläche der Kapsel (10);
Bereitstellen einer Texturiereinrichtung (20);
Anordnen der Texturiereinrichtung (20) über dem Material (18) und dadurch Einprägen einer Texturoberfläche in das Material;
Entfernen der Texturiereinrichtung (20) von dem Material;
Aushärten des Materials (18) zu der Kapsel (10);
Verschließen der Kapsel (10);
Füllen der Kapsel (10) mit einem Füllmaterial und Verschließen der ausgefüllten Kapsel, damit das Füllmaterial in der Kapsel bleibt.

2. Verfahren nach Anspruch 1, dadurch gekennzeichnet, daß die Haltevorrichtung eine im wesentlichen runde Scheibe (12) mit einem im wesentlichen flachen Querschnitt aufweist, die Scheibe eine erste Seite (14) und eine zweite Seite (16) hat und wobei die Kapsel (10) im wesentlichen auf der ersten Seite (14) der Scheibe (12) angeordnet ist.

3. Verfahren nach Anspruch 1 oder 2, dadurch gekennzeichnet, daß die Texturiereinrichtung (20) über dem Material (18) nur auf der ersten Seite (14) angeordnet ist.

4. Verfahren nach Anspruch 2, dadurch gekennzeichnet, daß das Material (18) an der Kapsel (10) auf der ersten Seite (14) und auf der zweiten Seite (16) der Scheibe (12) angeordnet ist und die Texturiereinrichtung (20) zwei texturierende Medien aufweist, von denen jeweils eines auf jeder Seite des Materials (18) der Scheibe (12) angeordnet ist.

5. Verfahren nach einem der Ansprüche 1 bis 4, dadurch gekennzeichnet, daß, nachdem das texturierende Medium (20) auf dem Material (18) angeordnet wurde, die Kapsel (10) zwischen einem Plattenpaar (22a, 22b) gepreßt wird.

6. Verfahren nach Anspruch 5, dadurch gekennzeichnet, daß die Platten (22a, 22b) Umgebungstemperatur aufweisen.

7. Verfahren nach Anspruch 5, dadurch gekennzeichnet, daß zumindest eine der Platten eine höhere Temperatur aufweist.

8. Verfahren nach einem der Ansprüche 1 bis 7, dadurch gekennzeichnet, daß die Haltevorrichtung ein Formkern (41) ist, der in der Form des Implantats geformt ist.

9. Verfahren nach einem der Ansprüche 1 bis 8, dadurch gekennzeichnet, daß das formbare, biologisch verträgliche Material aus Silikon besteht.

10. Verfahren nach Anspruch 9, dadurch gekennzeichnet, daß das Silikon aus einer Silikonplatte besteht.

11. Verfahren nach einem der Ansprüche 1 bis 10, dadurch gekennzeichnet, daß die Texturiereinrichtung (20) aus Schaum besteht.

12. Verfahren nach Anspruch 11, dadurch gekennzeichnet, daß der Schaum aus vernetztem Schaum besteht.

13. Verfahren nach einem der Ansprüche 1 bis 12, dadurch gekennzeichnet, daß die Texturiereinrichtung (20) aus einer texturierten Platte besteht.

14. Verfahren nach einem der Ansprüche 1 bis 12, dadurch gekennzeichnet, daß das texturierende Medium ein Raster aufweist.

15. Verfahren nach einem der Ansprüche 1 bis 14, dadurch gekennzeichnet, daß der Lappen (62) aus texturiertem Material besteht.

16. Verfahren nach einem der Ansprüche 1 bis 15, dadurch gekennzeichnet, daß der Schritt des Verschließens die Schritte aufweist:
Bereitstellen eines Lappens (62) aus teilweise vulkanisiertem Silikon;
Anordnen einer Texturiereinrichtung (67) über diesem;
Einsetzen des texturierten Lappens in ein Verschlußloch der Kapsel, wobei dessen texturierter Teil nach außen gerichtet ist;
Ankleben des Lappens an die Kapsel (60) mittels Silikon; und
Aushärten des Kapsellappens.

## Revendications

1. Procédé pour fabriquer un implant chirurgical texturé, comprenant les étapes suivantes :
procurer une coque de prothèse à surface lisse (10) ayant une surface extérieure;
disposer ladite coque (10) sur un moyen de montage (12);
disposer un matériau biocompatible formable (18) sur ladite coque au moins sur une portion de ladite surface extérieure;
procurer un moyen de texturation (20);
disposer ledit moyen de texturation (20) sur ledit matériau (18), imprimant ainsi dans ledit matériau une surface texturée;
retirer dudit matériau ledit moyen de texturation (20);
durcir ledit matériau (18) dans ladite coque (10);
poser une pastille sur ladite coque (10);
remplir ladite coque (10) avec un matériau de remplissage et poser une pastille sur ladite coque remplie pour retenir dans ladite coque ledit matériau de remplissage.

2. Procédé selon la revendication 1, caractérisé en ce que ledit moyen de montage comprend un disque pratiquement rond (12) ayant une section transversale pratiquement plane, ledit disque ayant un premier côté (14) et un deuxième côté (16), ladite coque (10) étant pratiquement disposée sur ledit premier côté (14) dudit disque (12).

3. Procédé selon la revendication 1 ou la revendication 2, caractérisé en ce que ledit moyen de texturation (20) est disposé sur ledit matériau (18) seulement sur ledit premier côté (14).

4. Procédé selon la revendication 2, caractérisé en ce que ledit matériau (18) est disposé sur ladite coque (10) sur ledit premier côté (14) et ledit deuxième côté (16) du disque (12), et en ce que ledit moyen de texturation (20) comprend deux moyens de texturation, un disposé sur ledit matériau (18) de chaque côté du disque (12).

5. Procédé selon l'une quelconque des revendications 1 à 4, caractérisé en ce que, après que le moyen de texturation (20) a été disposé sur ledit matériau (18), ladite coque (10) est comprimée entre deux plateaux (22a, 22b).

6. Procédé selon la revendication 5, caractérisé en ce que lesdits plateaux (22a, 22b) sont a la température ambiante.

7. Procédé selon la revendication 5, caractérisé en ce qu'au moins l'un desdits plateaux est à une température élevée.

8. Procédé selon l'une quelconque des revendications 1 à 7, caractérisé en ce que ledit moyen de montage est un mandrin (41) conformé à la forme dudit implant.

9. Procédé selon l'une quelconque des revendications 1 à 8, caractérisé en ce que ledit matériau compatible formable comprend un silicone.

10. Procédé selon la revendication 9, caractérisé en ce que ledit silicone est une feuille de silicone.

11. Procédé selon l'une quelconque des revendications 1 à 10, caractérisé en ce que ledit moyen de texturation (20) comprend une mousse.

12. Procédé selon la revendication 11, caractérisé en ce que ladite mousse est une mousse réticulée.

13. Procédé selon l'une des revendications 1 à 12, caractérisé en ce que ledit moyen de texturation (20) comprend une plaque texturée.

14. Procédé selon l'une quelconque des revendications 1 à 12, caractérisé en ce que ledit moyen de texturation comprend un écran.

15. Procédé selon l'une quelconque des revendications 1 à 14, caractérisé en ce que ladite pastille (62) comprend un matériau texturé.

16. Procédé selon l'une quelconque des revendications 1 à 15, caractérisé en ce que l'étape de pose de la pastille comprend les étapes suivantes :
procurer une pastille (62) de silicone partiellement vulcanisé;
disposer sur elle un moyen de texturation (67);
insérer ladite pastille texturée dans un trou de pastille de ladite coque avec sa portion texturée en vis-à-vis de l'extérieur;
faire adhérer ladite pastille sur ladite coque (60) avec un silicone; et
durcir la coque pourvue de la pastille.
